# EUROPEAN PATENT SPECIFICATION

(11) **EP 0 821 588 B1**
(45) Date of publication and mention of the grant of the patent: **18.06.2003**
(21) Application number: 96910087.4
(22) Date of filing: 15.04.1996
(51) Int. Cl.: A61K 31/445, A61P 23/02

(54) **LEVOBUPIVACAINE AND ITS USE AS AN ANAESTHETIC IN PREGNANT WOMEN**
LEVOBUPIVACAIN UND DESSEN VERWENDUNG ALS ANÄSTHETIKUM BEI SCHWANGEREN FRAUEN
LEVOBUPIVACAINE ET SON UTILISATION COMME ANESTHESIQUE CHEZ LA FEMME ENCEINTE

(30) Priority: 13.04.1995 GB 9507677; 27.10.1995 US 549408
(43) Date of publication of application: 04.02.1998
(73) Proprietor: Darwin Discovery Limited, Slough Berks SL1 3WE (GB)
(72) Inventor: BARDSLEY, Hazel, Judith, Milton Road Cambridge CB4 4WE (GB); GRISTWOOD, Robert, William, Milton Road Cambridge CB4 4WE (GB)
(74) Representative: Perry, Robert Edward
(86) International application number: GB9600912
(87) International publication number: WO96032109

(56) References cited:
- WO-A-95/10276
- WO-A-95/10277
- INTERNATIONAL JOURNAL OD OBSTETRIC ANALGESIA, vol. 4, no. 2, 1995, pages 93-108, XP000613009 REYNOLDS, F.: "In defence of Bupivacaine"
- REGIONAL ANAESTHESIA, vol. 17, 1992, pages 311-316, XP000610923 DENSON, D.D. ET AL: "Enantiomer-specific effects of an intravenously administered arrhythmogenic dose of bupivacaine on neurons of the nucleus tractus solitarius and the cardiovascular system in the anesthetized rat" cited in the application
- EXPERT OPINION ON INVESTIGATIONAL DRUGS, vol. 3, no. 11, November 1994, pages 1209-1212, XP000610836 GRISTWOOD, R. ET AL: "Reduced cardiotoxicity of levobupivacaine compared with racemic bupivacaine (Marcaine): new clinical evidence" cited in the application
- BRITISH JOURNAL OF PHARMACOLOGY, vol. 103, 1991, pages 1275-1281, XP000610827 VANHOUTTE, F. ET AL: "Stereoselective effects of the enantiomers of bupivacaine on the electrophysiological properties of the guinea-pig papillary muscle" cited in the application
- CHIRALITY, vol. 5, no. 7, 1993, pages 485-491, XP000610881 RUTTEN, A.J. ET AL: "Tissue distribution of bupivacaine enantiomers in sheep"
- ACTA PHARMACOLOGICA ET TOXICOLOGICA, vol. 31, 1972, pages 273-286, XP000610878 ABERG, G.: "Toxicological and local anaesthetic effects of optically active isomers of two local anaesthetic compounds"
- FEDERATION PROCEEDINGS, vol. 40, no. 31, 1981, page 684 XP002020663 KUHNERT, B.R. ET AL: "Bupivacaine disposition in mother, fetus and neonate"

## Description

### Field of the Invention

This invention relates to a new therapeutic use for levobupivacaine or (S)-1-butyl-N-(2,6-dimethylphenyl)-2-piperidinecarboxamide.

### Background of the Invention

Racemic bupivacaine is an effective long-acting local anaesthetic, and may be given as an epidural. However, racemic bupivacaine is cardiotoxic, having depressant electrophysiological and mechanical effects on the heart. It should therefore be used with caution in cardiac-compromised patients, and the use of high doses and high concentrations is contraindicated.

In particular, bupivacaine has produced death in a number of patients, including women in childbirth and when used in the Bier's block technique. Although the incidence of death has been relatively small, the concern has been sufficient to stop the use of 0.75% bupivacaine for obstetrics and the proscribing of bupivacaine for use in Bier's blocks.

In addition, due to its mode of action, directly on the nervous system, at higher doses, bupivacaine is known to have undesirable central nervous system (CNS) side-effects which, *prima facie*, are connected to its anaesthetic activity. Indeed, the occurrence of CNS side-effects is one of the major factors limiting the use of this drug in normal clinical practice employing techniques such as local infiltration, nerve block, field block, epidural and spinal blocks.

It has been suggested that levobupivacaine is less cardiotoxic than dexbupivacaine and racemic bupivacaine. See, for example, Vanhoutte *et al*, Br. J. Pharmacol. 103:1275-1281 (1991), and Denson *et al*, Regional Anaesthesia, 17:311-316 (1992). However, these reports are based on work *in vitro*, and cannot necessarily be extrapolated to any mammals, and certainly not to humans.

The effective utility of levobupivacaine in man, *in vivo,* is evidenced for the first time in WO-A-9510276, WO-A-9510277 and Gristwood *et al*, Exp. Opin. Invest. Drugs 3(11):1209-12 (1994). The latter documents indicate the potential utility of levobupivacaine in obstetrics, in part at least because of reduced CNS side-effects.

### Summary of the Invention

Surprisingly, it has now been found that while levobupivacaine retains the anaesthetic activity of the racemate, its utility in obstetrics is enhanced because of data showing greatly reduced uptake of levobupivacaine by the foetus. This unexpected discovery means that the mother can benefit from the use of the drug, if appropriate at relatively high concentration, without the unborn child being so much affected. This is relevant not only in obstetrics as such but also when anaesthetic is required during pregnancy. In addition, while levobupivacaine can be used at doses conventionally used for the racemic drug, it is now evident that it can also be used at higher doses and/or over longer periods, formerly contraindicated for the racemic drug, enabling better anaesthesia, e.g. in terms of availability to different patient types, extent of anaesthetic block achieved etc, without the adverse effects conventionally associated with these dose regimens.

According to a first aspect of the present invention, levobupivacaine is useful for providing anaesthesia in a pregnant woman, prior to delivery, e.g. in surgery.

### Description of the Invention

As indicated above, levobupivacaine is useful where it is desired to reduce CNS side-effects. These effects include tinnitus, numb tongue or lips, and dry mouth, and are used as early indicators of direct nervous system effects. For instance, CNS side-effects are typically used as warnings of the onset of convulsions (which in a pregnant woman may also be induced *in utero*) which must be avoided because of the risk to the patient, eg. death, brain damage, foetal distress etc. As a result, clinical administration of a local anaesthetic is stopped upon onset of these early symptoms, whether or not adequate anaesthesia or analgesia has been achieved. The dose at which CNS side-effects appear varies greatly between patients and cannot be predicted reliably.

According to the present invention, levobupivacaine may be provided in solution, for infusion or injection into the epidural or spinal space, or for administration by any of the conventional means for obtaining a nerve or field block.

Administration of levobupivacaine may be continuous or bolus administration. This may be done using conventional apparatus, e.g. including means for the patient to induce infusion as desired. The daily dose administered to the patient may be in the relatively low range known for the administration of racemic bupivacaine. Nevertheless, because of the decreased CNS side-effects of levobupivacaine, and its reduced effect on the foetus in pregnant women, the dosage may be higher than the conventional dose for the racemic drug. For instance, the patient may receive a daily dose of levobupivacaine of up to 2500 mg. However, it is preferred to provide a considerable safety margin for the patient, and therefore for the patient to receive a daily dose of less than 2000 mg. Consequently, the total dose of levobupivacaine may be around, or in excess of, 2 mg per kg of patient body weight.

The concentration of levobupivacaine to be given can be that conventionally used for the racemic drug. However, the concentration is typically higher than this, for instance, at least 0.75% w/v, and can be up to 2% w/v. Preferably, however, the concentration of levobupivacaine is in the range 0.8% to 1.5% w/v, and more preferably a concentration of 1%, 1.25% or 1.5% w/v is used. The solution is preferably aqueous.

The solution may typically be put up in unit doses of from 1 to 15 ml, and preferably of around 10 ml. However, the unit doses may be higher, for instance up to 40 ml or higher. The unit doses may be in the form of ampoules, which may be made of any suitable material, e.g. glass or an impervious plastics material. Unit dosages comprising at least 75 mg, but preferably less than 200 mg, of levobupivacaine can be administered, and more preferably the unit dosage is in the range 80 to 150 mg. Consequently, the patient may receive a daily dose of levobupivacaine of up to 2500 mg, but it is preferred that the daily dose is less than 2000 mg.

Another suitable formulation of levobupivacaine of high concentration is for topical administration, e.g. a depot gel.

The administration of levobupivacaine over a range of concentrations, including those currently used for the racemic drug and the higher concentrations described above, can be carried out for significantly longer periods than at present. For instance, levobupivacaine can be administered to a patient safely for at least 24 hours, often up to 72 hours, and even for periods of up to a week or a fortnight, or longer. It can, of course, be administered for similar periods already used for the racemic drug, eg. between 3 and 6 hours.

Medicaments according to the present invention are particularly useful in surgical procedures carried out on patients who are cardiac or CNS-compromised, or patients predisposed to cardiac or CNS-related conditions, i.e. having a low CNS threshold. Alternatively, the patient may be one in which the direct nervous system effects following CNS side-effects are particularly dangerous, or even lethal, e.g. a pregnant woman. The subject may be undergoing surgery, related or unrelated to pregnancy. Alternatively, the subject may be in pain, e.g. from arthritis.

The levobupivacaine used in the present invention is preferably substantially free of dexbupivacaine, and is more preferably in at least 90%, and most preferably at least 99%, enantiomeric excess with respect to dexbupivacaine. Throughout this specification, reference to bupivacaine and its enantiomers includes pharmaceutically-acceptable salts thereof.

The following provides the evidence that is the basis of the present invention. It comprises the procedures and results of a study into the effects on uterine blood flow (UBF) of two local anaesthetics, levobupivacaine (LB) and ropivacaine (R), in comparison with those of racemic bupivacaine (B), currently the most commonly used local anaesthetic in obstetric anaesthesia.

Thirty chronically-instrumented ewes, near term of pregnancy, were studied under an approved protocol. Animals were randomised to receive a two-step IV infusion of LB, R, or B at a rate of 0.07 mg/kg/min for 15 min followed by 0.035 mg/kg/min for 45 min. The rate of infusion was chosen to achieve serum concentrations of drug similar to those expected to occur during uneventful epidural anaesthesia for C/S. The investigators were blinded to the identity of the drug.

UBF was measured using a pulse transit-time Doppler flow probe. Measurements were made prior to (time 0) and at 30 and 60 min of infusion. Intraamniotic pressure was monitored continuously through an indwelling catheter. Serum drug concentrations were determined by gas chromatography in maternal and fetal arterial blood samples obtained at the end of infusion. Repeated measures ANOVA was used to detect statistically significant differences *p < 0.05. Results = mean ± SD.

Ten ewes were studied in each drug group. The maternal serum concentrations of LB, R and RB were 1.33±0.65, 1.17±0.41 and 1.56±0.61 µg.ml⁻¹, respectively, and the fetal concentrations were 0.20±0.15, 0.51±0.5 and 0.61±0.71, µg.ml⁻¹. Therefore, the corresponding F/M ratios of serum concentrations were 0.20±0.3, 0.41±0.35 and 0.51±0.60. This reflects a reduced transfer of levobupivacaine to the fetus.

No drug resulted in a significant change in UBF or intraamniotic pressure.

| UBF (ml/min) | | | |
|---|---|---|---|
| Time | LB | R | B |
| 0 | 457 ± 139 | 451 ± 118 | 466 ± 121 |
| 30 | 453 ± 127 | 453 ± 119 | 472 ± 113 |
| 60 | 450 ± 136 | 467 ± 119 | 469 ± 127 |

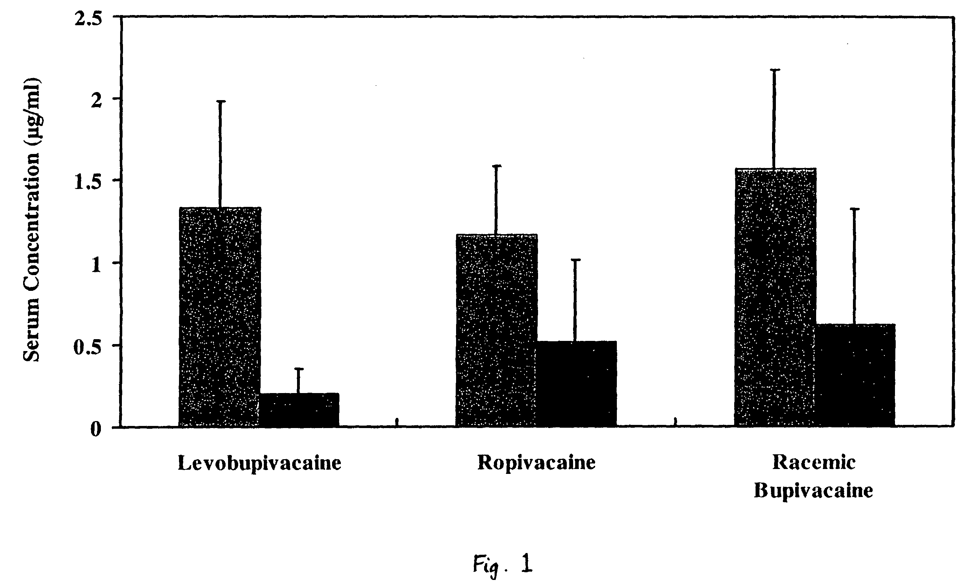

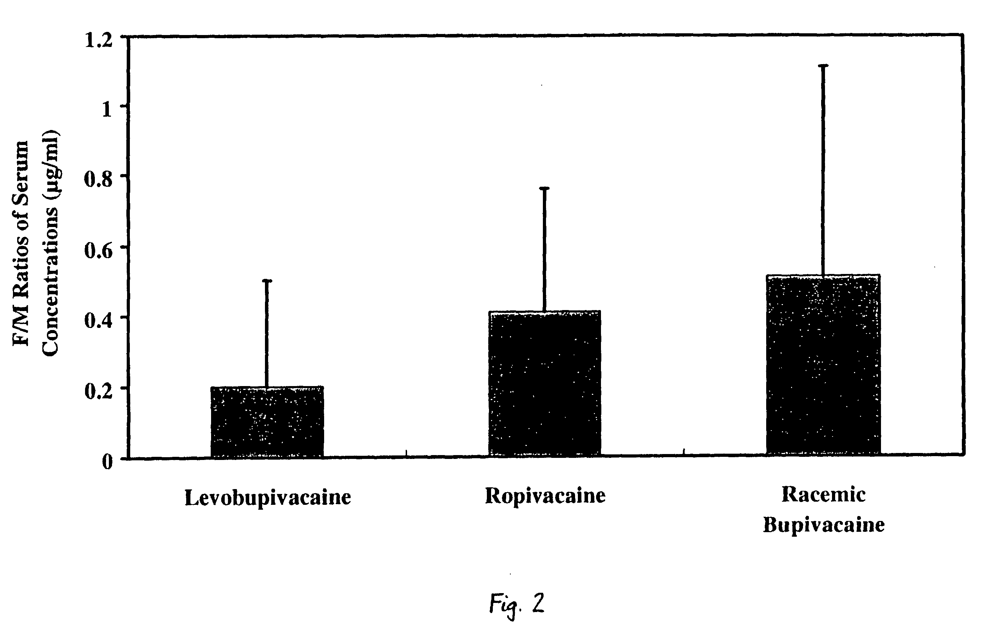

The dramatic differences between the results for the anaesthetic tested are best illustrated by the accompanying drawings, which reproduce the results shown above. More particularly, Fig. 1 shows the mean maternal (higher, lefthand column in each pair) and fetal (lower, right-hand column in each pair) serum concentrations at the end of drug infusion (±SD). Fig. 2 shows the mean F/M (fetal/maternal) ratios of serum concentrations (±SD).

The composition of the present invention is illustrated in the following Example.

### Example

A sterile isotonic aqueous solution of levobupivacaine was made up using the following components:
1.00 g levobupivacaine hydrochloride (measured as the free base)
0.9 g sodium chloride
to 100 ml water for injection

The solution was made up under sterile conditions (alternatively, it could have been sterilised after make-up, e.g. by sterile filtration).

10 ml aliquots of the solution were filled into sterilised glass ampoules, which were then sealed ready for use.

## Claims

1. Use of levobupivacaine for the manufacture of a medicament for use in providing anaesthesia in a pregnant woman, prior to delivery.

2. Use according to claim 1, wherein the medicament is an aqueous solution.

3. Use according to claim 1 or claim 2, wherein the levobupivacaine is present in at least 90% enantiomeric excess with respect to dexbupivacaine.

## Patentansprüche

1. Verwendung von Levobupivacain zur Herstellung eines Arzneimittels zur Verwendung bei der Anästhesie einer schwangeren Frau vor der Entbindung.

2. Verwendung nach Anspruch 1, wobei das Arzneimittel in Form einer wässrigen Lösung vorliegt.

3. Verwendung nach Anspruch 1 oder 2, wobei das Levobupivacain in einem mindestens 90%igen enantiomeren Überschuss im Verhältnis zu Dexbupivacain vorliegt.

## Revendications

1. Utilisation de lévobupivacaïne pour la fabrication d'un médicament pour provoquer une anesthésie de la femme enceinte, avant l'accouchement.

2. Utilisation selon la revendication 1, dans laquelle le médicament est une solution aqueuse.

3. Utilisation selon la revendication 1 ou 2, dans laquelle la lévobupivacaïne est présente en excès énantiomérique d'au moins 90 % par rapport à la dextrobupivacaïne.
